# EUROPEAN PATENT APPLICATION

(11) **EP 3 682 890 A1**
(43) Date of publication of application: **22.07.2020**
(21) Application number: 20152389.1
(22) Date of filing: 17.01.2020
(51) Int. Cl.: A61K 35/741, A61K 9/00, A61K 35/744, A61K 35/745, A61K 35/747, A61P 15/02, A61P 17/00, A61P 31/10, A61Q 5/00, A61Q 19/00

(54) **COMPOSITION COMPRISING NON-VIABLE PROBIOTIC BACTERIA AND ITS USE IN THERAPY**

(30) Priority: 18.01.2019 IT 201900000801
(71) Applicant: Proge Farm S.r.l., 28100 Novara (NO) (IT)
(72) Inventor: MALFA, Patrizia, 28100 Novara NO (IT); DONDI, Giancarla, 28100 Novara NO (IT)
(74) Representative: Trupiano, Federica

(57) **Abstract**

The present invention refers to compositions, comprising non-viable probiotic bacteria, and their use in therapy, in particular, but without limitation, of the topical compositions, which are useful in the treatment of skin and mucosa infections as well as of allergies.

## Description

### ABSTRACT

The present invention refers to compositions, comprising non-viable probiotic bacteria, and their use in therapy, in particular, but without limitation, topical compositions, which are useful in the treatment of skin and mucosa infections as well as allergies.

### STATE OF THE ART

It has been recently observed that diseases caused by the proliferation of pathogenic microorganisms on the skin and on the mucosae can be treated by the topical use, i.e. directly on the affected area, of non-pathogenic bacteria, such as for example the probiotic bacteria, which colonize the skin or mucosa portion on which they are applied, thus hindering and often completely solving the ongoing infection.

The treatment or prophylaxis of said skin diseases by the topical use of probiotic bacteria has some advantages with respect to the conventional topical treatment with antimicrobials; e.g., by using the latter under certain conditions, the well-known antimicrobial resistance effect can occur.

The topical compositions comprising probiotic bacteria proved to be useful not only for the skin diseases caused by pathogenic microorganisms, such as *Malassezia* or *Candida* spp., but it has been observed that they allow to prevent and treat various skin and mucosal diseases, such as for example dandruff, various kinds of dermatitis, candidiasis and other related diseases such as, for example, endometriosis.

It has been also observed that some probiotic bacteria are able to prevent and treat skin diseases of allergic type and exercise immunostimulant action, such as for example described in the European Patent EP1915058B1, by the same Applicant. In particular, this patent describes the use of two strains of *Lactobacillus* for the preparation of an oral composition, wherein said viable bacteria provide a positive action in the treatment and prevention of the diseases related to alterations of the immune system and allergic diseases. To carry out in-vitro tests to demonstrate such activity, it has been necessary to inhibit the growth of the tested bacteria since the latter would have invalidated the development of the lymphocyte population and, as a consequence, would have altered the ratio between bacteria and lymphocytes which is an index of the proliferative response. However, no direct teaching of the use of said inactivated bacteria for any therapeutic object is described in such document.

In the past, most of the probiotic bacteria were usually administered in viable form or in the form of lysate.

Some international regulations have recently resolute that the biological material, including the probiotic bacteria, must be inactivated, i.e. made "non-viable", before its administration to humans and animals.

Therefore, most of the probiotic bacteria are now currently subjected to thermal treatments, such as for example the "tyndallization", said treatments being capable of inactivating the bacteria, making them unable to reproduce itself.

Even if it is a quite effective technique in order to inactivate said bacteria, tyndallization is a long and expensive process causing damages to the bacterial wall thus compromising adhesion abilities. There are other treatments which can inactivate said bacteria such as, for example, the lysis or the spray-dry treatment; in these cases, the results are fragments of "non-viable" bacterium where the wall is completely destroyed by the treatment.

There is therefore the need of overcoming the drawbacks of such techniques and thus providing novel inactivation processes which allow the bacterial cells to remain intact in order to show the adhesines on the surface, which are proteins involved in the adhesion mechanism of probiotic bacteria suitable for the administration to humans and animals.

### OBJECTS OF THE INVENTION

Object of the present invention is to provide a composition which comprises non-viable probiotic bacteria, to be essentially used topically.

Object of the present invention is also to provide the use of said topical composition in the prophylaxis and treatment of skin, mucosa and immune system diseases. Another object of the invention is to provide a composition comprising non-viable probiotic bacteria, to be essentially used topically, said bacteria being made non-viable by means of techniques which exclude the thermal treatment, such as tyndallization. These and other objects will become clear from the following specification and from the appended claims.

### BRIEF DESCRIPTION OF THE FIGURES

Figure 1 shows the result on the bacterial viability of the gamma ray inactivation treatment.
Figure 2 shows the results of the *in vitro* adhesion test carried out on viable and non-viable strains of *Lactobacillus plantarum* P17630. On the ordinate the number of adhered bacteria per VEC cell is depicted.
Figure 3 shows the results of the *in vivo* adhesion test to the vaginal epithelium of rabbits carried out on non-viable strains of *Lactobacillus plantarum* P17630 according to the invention. On the ordinate the number of CFU (colony-forming units) found in the histological sample of tissue analyzed following the animal sacrifice is depicted.

### DESCRIPTION OF THE INVENTION

The subject matter of the invention, according to one of the aspects thereof, is a composition, preferably a topical composition, comprising at least one strain of at least one inactivated probiotic bacterium, said bacterium having the cell wall substantially intact.

In particular, subject of the invention is a topical composition comprising at least one strain of at least one probiotic bacterium inactivated by the impairment of the DNA functionality, said bacterium having the cell wall substantially intact.

The term "impairment of the DNA functionality" denotes herein that the above mentioned probiotic bacterium has undergone an inactivation process which damaged the DNA structure of said bacterium, in any possible way, e.g. by causing breaks in the nucleotide chains, denaturing the DNA double helix, etc. Such lesion does not allow the bacterium to carry out the gene transcription and to form the proteins necessary to the life of the same bacterium, making it unable to metabolize, grow and reproduce itself.

According to the present invention, the term "inactivated probiotic bacterium" (or even only "non-viable bacterium" or "bacterium according to the invention" or variants thereof) therefore denote that said bacterium has been made "non-viable", by the impairment of its metabolism, its growth and its reproduction, by the lesion of its DNA.

According to the present invention, the expression "substantially intact cell wall" denotes that the wall of the bacterium is intact or in any case lesioned in a marginal way, and that it is still capable to adhere to the tissues of the subject to whom it is topically administered, as it will be discussed in more detail below.

In the present description, the non-viable probiotic bacterium according to the invention is also named "deactivated". Said deactivated bacterium it is not a bacterium subjected to thermal treatments, such as tyndallization.

According to a preferred embodiment, said at least one probiotic bacterium is selected from the bifidobacterium, lactobacillus, streptococcus genera and mixtures thereof.

According to a preferred embodiment, said at least one probiotic bacterium is selected from *Lattobacilli,* advantageously from *L. acidophilus, L. amylovorus, L. brevis, L. bulgaricus, L. casei, L. crispatus, L. delbrueckii, L.fermentum, L. gasseri, L. ingluviei, L. iners, L. intestinalis, L. jensenii, L. johnsonii, L. kalixensis, L. kefiranofaciens, L. kefiri, L. kimchii, L. kitasatonis, L. kunkeei, L. lactis, L. leichmannii, L. lindneri, L. malefermentans, L. mali, L. manihotivorans, L. mindensis, L. mucosae, L. murinus, L. nagelii, L. namurensis, L. nantensis, L. oligofermentans, L. oris, L. panis, L. pantheris, L. parabrevis, L. parabuchneri, L. paracasei, L. paracollinoides, L. parafarraginis, L. parakefiri, L. paralimentarius, L. paraplantarum, L. pentosus, L. perolens, L. plantarum, L. pontis, L. psittaci, L. rennini, L. reuteri, L. rhamnosus, L. rimae, L. rogosae, L. rossiae, L. ruminis, L. saerimneri, L. sake, L. salivarius, L. sanfranciscensis, L. satsumensis, L. secaliphilus, L. sharpeae, L. siliginis, L. spicheri, L. suebicus, L. thailandensis, L. trichodes, L. uli, L. ultunensis, L. vaccinostercus, L. vaginalis, L. versmoldensis, L. vini, L. vitulinus, L. zeae, L. zymae* and mixtures thereof.

According to a particularly preferred embodiment, said at least one probiotic bacterium is selected from
- *Lactobacillus salivarius* I 1794, *Lactobacillus paracasei* I 1688, *Lactobacillus paracasei* I 1687, *Lactobacillus fermentum* I 3677, all filed at the CIP Institut Pasteur of Paris, France, by the same Applicant;
- *Lactobacillus plantarum* P 17630, *Lactobacillus crispatus* P 17631, *Lactobacillus gasseri* P 17632, *Lactobacillus gasseri* P 18137, *Lactobacillus acidophilus* P 18806, *Lactobacillus delbrueckii* P 18805, *Streptococcus thermophilus* P 18807, *Lactobacillus brevis* CV7Lac (P30019), *Lactobacillus paracasei* LC11 (P30020), *Lactobacillus brevis* Y1 (P30021), *Lactobacillus brevis* CV2Lac (P30022), *Lactobacillus plantarum* CVI15B (P30023), *Lactobacillus paracasei* LC18 (P30024), *Lactobacillus plantarum* LM2 (P30025), all filed at the BCCM-LMG Laboratorium vor Microbiologie, University of Gent, Gent Belgium, by the same Applicant;
- *Lactobacillus rhamnosus* ATCC®53103™, filed at the American Type Culture Collection of Manassas, VA, USA;
and mixtures thereof.

According to a preferred embodiment, said at least one probiotic bacterium is selected from *Lactobacillus plantarum* P 17630, *Lactobacillus crispatus* P 17631, *Lactobacillus paracasei* I 1688, *Lactobacillus salivarius* I 1794 and mixtures thereof. According to a preferred embodiment, said at least one probiotic bacterium is selected from *Lactobacillus crispatus* P 17631, *Lactobacillus paracasei* I 1688, *Lactobacillus salivarius* I 1794 and mixtures thereof.

According to a preferred embodiment, said at least one probiotic bacterium is selected from *Lactobacillus plantarum* P 17630.

With "composition" it is meant, according to the present invention, any pharmaceutical, dietetic, food or nutraceutical composition, which comprises the non-viable bacteria described above or mixtures thereof.

According to a preferred embodiment, the composition of the invention is a topical composition.

The term "topical composition" (or its plural) denotes herein a pharmaceutically acceptable composition suitable for the application to the skin and/or mucosae of the human or animal, this last being preferably a mammalian.

The expression "probiotic bacterium" is conventionally used in the art and does not require specific explanations.

As mentioned, the non-viable probiotic bacterium of the invention is inactivated by one or more treatments suitable to compromise its metabolism, growth and reproduction, still keeping its cell wall substantially intact.

Said treatments exclude therefore the thermal treatments, such as for example the tyndallization.

According to a preferred embodiment, the non-viable probiotic bacterium of the invention is inactivated by the gamma-ray treatment, which damages its DNA and makes the bacterium unable to metabolize, grow and reproduce itself, but which dose not alter the cell wall of said bacterium.

The gamma-ray irradiation is a well-known technique which considers the exposure of a product, in this case at least one strain of at least one probiotic bacterium, to electromagnetic radiations, usually emitted by cobalt 60 (⁶⁰Co) or, more rarely, cesium 137 (¹³⁷Ce).

In practice, it is possible to irradiate one or more strains of probiotic bacteria, preferably in freeze-dried form, with gamma-rays in doses sufficient to inactivate said one or more strains of probiotic bacteria; by way of examples, a 10-50 kiloGrey (kGy), preferably a 20-40 kGy dosage can be used, until the full inactivation of said one or more strains of probiotic bacteria. The one skilled in the art can perfectly determine the parameters and the conditions necessary for said inactivation.

Moreover, the achieved full inactivation can be easily verified by plate counting, i.e. by seeding the so-treated bacteria on a suitable culture medium, according to procedures well-known to the one skilled in the art, and by controlling the lack of bacterial growth. By way of example, it is possible for example to plate seeding, under the same conditions, the corresponding non-irradiated strain, e.g. in an amount of 1x10⁸-10¹², e.g. 1x10¹¹ CFU/g (Colony-Forming Units/gram).

It is generally recognized that the bacterial adhesion to the skin or epithelial mucosa plays a key role, in any district, in the physiology of the natural microbiota in humans and in the pathogenesis of infections sustained by pathogenic bacteria, even if neither the mechanism nor the factors regulating the adhesion have been yet fully understood.

Some authors claim that a key role in the adhesion mechanism is played by chemical bonds between surfaces; others report that the carbohydrates layers, covering the bacterial cells, mediate the fixation; still others predict the presence of particular molecules and/or molecular and/or macromolecular structures responsible for the adhesion, such as the complex macromolecules generally identified as "adhesines".

It is known that probiotic bacteria have shown marked adhesion ability to epithelial cells such as those making up the skin, the vaginal mucosa, the intestinal mucosa, etc. In the literature, studies are reported demonstrating how the different adhesion ability is typical of a single subject and not of the full bacterial species since, for the purposes of the adhesion mechanism, both the surface nature on which the microorganism will have to adhere and above all the intrinsic characteristics of the microorganisms itself are important.

In the European Patent EP1046713B1, by the present Applicant, it is described a method to select lactic acid bacteria strains with particular adhesive abilities for the human cells and tissues. In said patent it is also clearly described the *Lactobacillus plantarum* P 17630, a bacterial strain which showed marked adhesive properties which allow it to prevent and even to compete with the pathogenic microorganisms thanks to the lactic acid bacteria biofilm which it can form. This biofilm is a real barrier between the epithelium and possible pathogenic bacteria, since it avoids the adherence of pathogens to the tissue surface of the epithelial cells, this way preventing and/or opposing the toxicogenic infections.

It has been observed that, even if not viable, the irradiated probiotic bacteria according to the invention retain their adhesive properties, being the cell wall not affected by the treatment. Said bacteria maintain therefore the molecules present on their external wall, such as for example the "adhesines", and can play their beneficial action on the skin and mucosae.

The non-viable bacterium of the invention is preferably a bacterium which has good adhesive abilities to the cells and tissues of the topically administered subject, said subject being advantageously a mammalian, preferably a human being.

According to a particularly preferred embodiment, the non-viable bacterium of the invention is selected for its adhesive abilities, advantageously according to the method described in the Patent EP1046713B1. Advantageously, said non-viable bacterium is the *Lactobacillus plantarum* P 17630.

As already mentioned, the compositions of the invention are useful for the prevention and/or the treatment of skin and mucosal diseases in general, e.g. for various kinds of dermatitis, such as atopic dermatitis, allergic contact dermatitis and irritative contact dermatitis, urticaria, erythema, dandruff, lichen planus, psoriasis, aphthae and the like.

Moreover the compositions of the invention are particularly useful for the prevention and/or the treatment of diseases caused by the proliferation of pathogenic microorganisms, e.g. bacteria and fungi; such diseases including pityriasis versicolor, seborrheic dermatitis, dandruff caused by fungi of the *Malassezia* genus, tinea pedis or athlete's foot, tinea nigra, white trichosporosis, black trichosporosis, pityrosporum folliculitis, dermatomycosis, candidiasis, onychomycosis, impetigo, leishmaniasis, scabies, aphthae and the like.

Moreover, thanks to their adhesive abilities, the compositions of the invention can be used also as immunostimulants of the skin and mucosae and can play an advantageous action against allergies.

According to one of the preferred embodiments, the composition of the invention is particularly suitable for the vaginal administration, to oppose disorders such as infections caused by pathogenic microorganisms. In this case, the composition of the invention can be suitably formulated in vaginal suppositories or capsules, e.g. in hard or soft gelatin capsules, or still in gel, pessaries, creams and the like.

According to an embodiment, the topical compositions of the invention comprise at least one strain of at least one probiotic bacterium made non-viable according to the invention, together with conventional excipients, including water and salines and, if desired or necessary, together with other possible active ingredients. By way of example, pharmaceutically acceptable metal or semimetal oxides can be added to said compositions.

The topical composition of the invention can be any conventional topical composition, formulated and/or prepared by means of equally known processes. For example, the topical composition of the invention can be selected from: creams, multiple emulsions such as oil and/or silicone in water emulsions, water-in-oil and/or -silicone emulsions, water/oil/water or water/silicone/water emulsions and oil/water/oil or silicone/water/silicone emulsions, anhydrous compositions, aqueous dispersions, oils, milk, balms, foams, lotions, gel, creme gel, hydroalcoholic solutions, hydroglicolic solutions, hydrogel, liniments, waxes, soaps, shampoos, emollients, serums, unguents, mousses, ointments, powders, pastes, spray and the like. The composition can be also administered by means of polysaccharide films, sticking patches, non-sticking patches, occlusive patches and micro-electric patches.

Alternatively, the composition of the invention can be incorporated into a solid accessory, e.g. selected from bandages, bands, gauzes, t-shirts, shocks, tights, linen, belts, gloves, diapers, pads, dresses, bedcovers, wipes, facial masks; the incorporation of the composition of the invention into a solid accessory allows the ease of application of the composition of the invention, and moreover, in some embodiments, such as for example when the composition of the invention is incorporated into t-shirts, allows to the same to adhere over a wide body surface, which is useful when large area of the skin have to be treated, such as the back, e.g. to prevent and/or treat the pityriasis versicolor.

Alternatively, the composition can be in the form of cosmetic products such as deodorants, sun products, moisturizing creams, anti-aging creams, anti-pollution creams, creams containing pigments, radiant creams, colored and non-colored powders for face and body, cream foundation, makeup remover lotions, makeup remover milks, concealers, mascara, eye shadows, lipsticks, lip balms, lip glosses and the like. Said incorporation can be carried out by means of conventional methods, well-known to the one skilled in the art.

The composition of the invention can be comprised or incorporated into single-dose containers or multi-dose containers. For example, the composition of the invention can be an unguent divided into single-dose containers, e.g. inside capsules, advantageously single-dose squeezable capsules.

In some cases, such as for example in the treatment of oral mucosa, but without limitation, a composition in the form of a gel can be suitable. Said composition in the form of gel is formulated according to the techniques well-known to the one skilled in the art and, in addition to the inactivated bacteria of the invention, it can for example comprise one or more of the following excipients and/or functional additives: hyaluronic acid, mallow extract, maltodextrins, sorbitol, sodium carboxymethyl cellulose, aloe vera extract, polyvinylpyrrolidone, carboxymethyl cellulose, sucralose, glyceryl monooleate, sesame oil, arabic gum and optional aromas.

The amount of the composition inside the single-dose container can vary as a function of the area to be treated. Accordingly, different single-dose containers containing different amounts of composition can be considered.

By way of example, a representative single-dose composition of the invention can contain an amount of the above described probiotic bacteria in the range from 1x 10¹ to 1x 10¹⁴ of at least one strain of at least one probiotic bacterium, preferably from 1x 10¹ to 1 x 10¹¹ bacteria, more preferably from 1x 10¹ to 1 x 10⁹ bacteria per gram. Unless otherwise stated, it is considered that, before the inactivation as described in the present invention, 1 gram of bacteria contains from about 1x 10¹ to 1x 10¹⁴ CFU/g (Colony-Forming Units/gram), preferably from 1x 10³ to 1x 10¹¹ CFU/g, more preferably from 1x 10⁵ to 1x 10¹⁰ CFU/g. It is clear that, after the inactivation according to the invention, preferably by means of gamma-rays, the bacterial content per gram will be essentially the same, but said bacteria will not be able to form colonies.

By way of example, the compositions of the invention contain from 1 mg to 2000 mg, e.g. from 10 to 1500 mg, preferably from 50 to 1000 mg, advantageously from 100 to 1000 mg, e.g. 50, 100, 200, 300, 500 or 1000 mg of non-viable bacteria, such dosage being clearly a function of the number of bacteria per gram.

The non-viable bacteria according to the invention are preferably included into the compositions of the invention in the freeze-dried form.

A preferred composition comprises the non-viable strain of *Lactobacillus plantarum* P 17630, e.g. in an amount of 10⁸-10¹⁰ CFU/g, said CFU being evaluated before the inactivation treatment.

According to a preferred embodiment, the composition of the invention is a composition for the vaginal administration, advantageously comprising the non-viable strains of *Lactobacillus plantarum* P 17630.

Representative examples of compositions according to the invention are set forth in the experimental section below.

In general, in order to exert the beneficial effect against the pathogenic microorganisms, the composition is applied on the skin and the mucosae 1 or more times per day, better on wet skin, being preferably applied over a wider area with respect to the visually affected area, in order to guarantee the coverage of the area affected by the disease. The treatment can last from 1 to 30 days, e.g. from 3 to 15 days or from 6 to 10 days. If necessary, the treatment can be further prolonged or periodically repeated.

It is a further object of the invention a method for the prevention and/or the treatment of the skin and mucosal diseases mentioned above, comprising topically administering an effective amount of the composition of the invention as illustrated above, to a subject in the need thereof.

Subject matter of the invention, according to another of the aspects thereof, is a pharmaceutical, dietetic, food or nutraceutical composition, comprising at least one non-viable probiotic bacterium selected from *Lactobacillus plantarum* P 17630, *Lactobacillus crispatus* P 17631, *Lactobacillus paracasei* I 1688, *Lactobacillus salivarius* I 1794 and mixtures thereof, said at least one bacterium having the cell wall substantially intact.

Subject of the invention, according to another of the aspects thereof, is a pharmaceutical, dietetic, food or nutraceutical composition, comprising at least one non-viable probiotic bacterium selected from *Lactobacillus plantarum* P 17630, *Lactobacillus crispatus* P 17631, *Lactobacillus paracasei* I 1688, *Lactobacillus salivarius* I 1794 and mixtures thereof, said at least one bacterium having the cell wall substantially intact and being made non-viable by means of techniques which exclude thermal treatments, such as tyndallization, preferably by means of the gamma-ray exposure technique.

Preferred and advantageous aspects of the invention set forth above with reference to the composition are considered applicable also to the composition and the method of treatment according to the invention.

Subject matter of the invention, according to another of the aspects thereof, is a strain of *Lactobacillus* selected from *Lactobacillus brevis* CV7Lac (P30019), *Lactobacillus paracasei* LC11 (P30020), *Lactobacillus brevis* Y1 (P30021), *Lactobacillus brevis* CV2Lac (P30022), *Lactobacillus plantarum* CVI15B (P30023), *Lactobacillus paracasei* LC18 (P30024), *Lactobacillus plantarum* LM2 (P30025), all filed at the BCCM-LMG Laboratorium vor Microbiologie, University of Gent, Gent Belgium, both in viable and inactivated form, having the cell wall substantially intact.

Subject of the invention, according to another of the aspects thereof, is a strain of *Lactobacillus* selected from *Lactobacillus brevis* CV7Lac (P30019), *Lactobacillus paracasei* LC11 (P30020), *Lactobacillus brevis* Y1 (P30021), *Lactobacillus brevis* CV2Lac (P30022), *Lactobacillus plantarum* CVI15B (P30023), *Lactobacillus paracasei* LC18 (P30024), *Lactobacillus plantarum* LM2 (P30025), for its use in the treatment of the diseases described herein, both in viable and inactivated form, as well as the compositions comprising it, e.g. as described herein.

The experimental section below is set forth for illustrative purpose only and not in any limitative way.

### EXPERIMENTAL SECTION

### Example 1

A soft gelatin capsule having the following quali-quantitative composition is prepared:

| | **mg/CAPSULE** |
|---|---|
| CONTENT | |
| Medium-chain triglycerides | 1523.60 |
| Non-viable *Lactobacillus plantarum* P 17630 (10⁹/g) | 100.00 |
| Lactose | 684.20 |
| Silicon dioxide | 47.00 |

| SHELL | |
|---|---|
| Hydroxypropyl starch from corn | 212.01 |
| Glycerol (E422) | 173.15 |
| Carrageenan (E407) | 68.59 |
| Titanium dioxide (E 171) | 8.13 |
| Disodium phosphate | 6.23 |

### Example 2

A soft gelatin capsule having the following quali-quantitative composition is prepared:

| | **mg/CAPSULE** |
|---|---|
| CONTENT | |
| Medium-chain triglycerides | 1302.00 |
| Non-viable *Lactobacillus plantarum* P 17630 (10⁹/g) | 100.00 |
| Lactose | 784.20 |
| Silicon dioxide | 62.00 |

| SHELL | |
|---|---|
| Hydroxypropyl starch from corn | 212.01 |
| Glycerol (E422) | 173.15 |
| Carrageenan (E407) | 68.59 |
| Titanium dioxide (E 171) | 8.13 |
| Disodium phosphate | 6.23 |

### Example 3

A hard gelatin capsule having the following quali-quantitative composition is prepared:

| | **mg/CAPSULE** |
|---|---|
| CONTENT | |
| Non-viable *Lactobacillus plantarum* P 17630 (10⁹/g) | 100.00 |
| Corn starch | 129.00 |
| Mannitol | 102.00 |
| Sodium croscarmellose (Crosslinked sodium carboxymethylcellulose) | 6.00 |
| Vegetable magnesium stearate | 4.00 |
| Hydrated colloidal silica (Silicon dioxide) | 3.00 |

| SHELL | |
|---|---|
| Titanium dioxide (E 171) | 1.83 |
| Hydroxypropyl methyl cellulose (hypromellose) | 93.17 |

### Example 4

A squeezable capsule having the following quali-quantitative composition is prepared:

| | **mg/CAPSULE** |
|---|---|
| CONTENT | |
| TOPLAC® Mixture: | 250.00 |
| Non-viable mixture of lactobacilli (4 x 10⁸/g) and modified metal oxides | |
| Medium-chain triglycerides | 948.44 |
| Silicon dioxide | 43.75 |
| Iron oxides | 0.71 |
| Fragrance | 7.50 |

| SHELL | |
|---|---|
| Modified starch E1440 | 45.5562 |
| Vegetable glycerol EP, USP, E422 | 35.1201 |
| Anhydrous disodium phosphate E339 | 1.1681 |
| Iota carrageenan E407 | 15.5748 |
| Mica | 1.5603 |
| Titanium dioxide | 0.7753 |
| Black iron oxide | 0.0384 |
| Red iron oxide | 0.2068 |

### Example 5

A soft gelatin capsule having the following quali-quantitative composition is prepared:

| | **mg/CAPSULE** |
|---|---|
| CONTENT | |
| Medium-chain triglycerides | 970.00 |
| Non-viable *Lactobacillus acidophilus* P 18806 (10⁹/g) | 100.00 |
| Silicon dioxide | 37.00 |

| SHELL | |
|---|---|
| Gelatin F.U. | 200.50 |
| Glycerol F.U. | 107.60 |
| Titanium dioxide E 171 | 6.90 |

### Example 6

### Process of gamma irradiation

A treatment of inactivation of a bacterial lyophilized product of 1x10¹¹ CFU/g of *Lactobacillus plantarum P 17630* has been carried out by gamma-ray irradiation under the following conditions:
Source: cobalt 60 (⁶⁰Co)
Nominal dose: 25 kGy

The process has been carried out at room temperature. The duration of the treatment has been calculated as a function of the activity of the power source used in order to administer the same preset nominal dose.

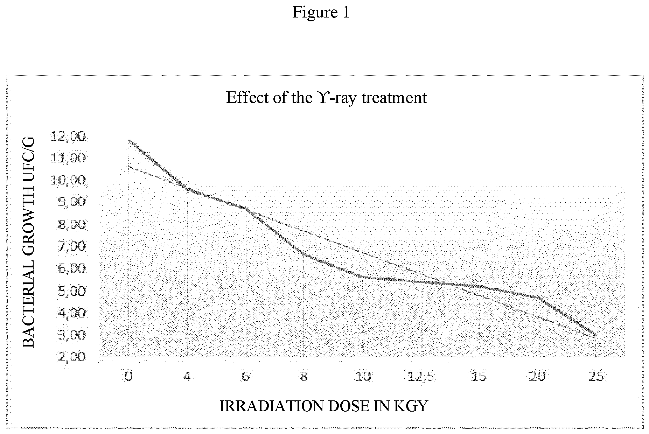

The result of the bacterial viability reduction at the different treatments is set forth in Figure 1.

### Example 7

### In vitro adhesion test

A comparison test, in duplicate, between the viable and the inactivated strain according to the invention has been prepared, in order to evaluate, *in vitro,* the adhesion ability to the vaginal epithelial cells of the *Lactobacillus plantarum* P17630 in viable form and in inactivated form according to Example 6.

The analyses for the two tests have been carried out under the same conditions, starting from vaginal epithelial cells (VEC) of different donors. Within the same test, the samples consisting of viable bacterial strains and non-viable bacterial strains (inactivated) have been prepared by reconstituting 1 gram of lyophilized product in 40 ml of Eagle Minimal Essential Medium adjusted to pH 5.50 (MEM). The bacterial suspension has been homogenized for 180 seconds, then washed with MEM. To have homogeneity in the evaluation, the preparation of the viable strain and that of the inactivated strain had the same initial concentration.

At the same time the vaginal epithelial cells (VEC) have been also prepared by resuspending a buffer in 5 ml of MEM and centrifuging at 1000 rpm to remove the possible endogenous microorganisms.

For the adhesion test, 500 µl of the VEC suspension have been co-incubated in a Petri dish with 500 µl of bacterial suspension obtained as described above. The dish has been incubated at 37°C for 30 minutes. The suspension has been filtered and the VEC cells have been harvested, fixed onto a slide, then stained.

The adhesion has been evaluated as the number of adhered bacterial cells, for a single VEC cell, by evaluating at least 10 VEC/sample.

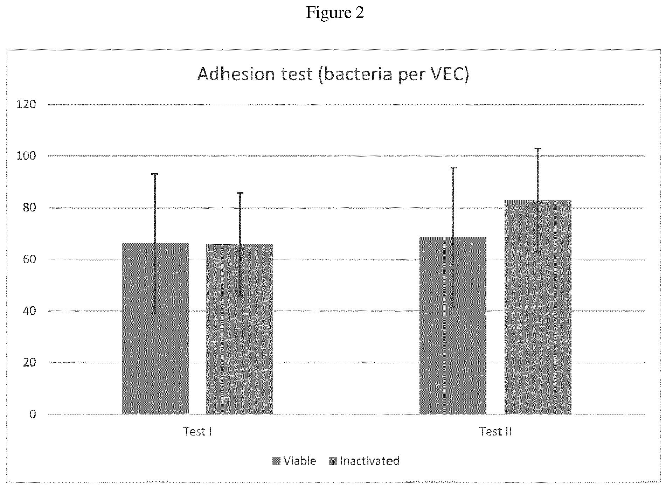

The results are depicted in the plot of Figure 2.

### Example 8

### In vivo adhesion test

A test has been prepared in triplicate to evaluate, *in vivo,* the adhesion ability to the vaginal epithelial cells of the inactivated *Lactobacillus plantarum* P17630, as well as the irritating effect on the vaginal mucosa according to the "Vaginal Irritation Test ISO 10993-10:2010".

The tests have been carried out on 3 groups of female albino rabbits (White rabbits, New Zealand, weight at the beginning of the test 2,075-2,200 g) and under the same conditions also on a control group.

In particular, the treated group (TG) has been vaginally administered once a day for five days with 1 ml of sample. Said sample is prepared starting from the soft capsules of Example 3 of the present experimental section (inactivated *Lactobacillus Plantarum* P17630, according to Example 6: Vaginal Capsule NV) by cutting the gelatin shell in small pieces and diluting the internal powder and the shell in a 0.9% sodium chloride solution in a ratio of 1:1.5 g/ml.

The control group (CG) has been treated with the same protocol (1 ml once a day for five days, vaginal route) by administering, instead of the sample containing the *Lactobacillus plantarum* P17630, 1 ml of a 0.9% sodium chloride solution.

All the treated animals have been controlled every 24 hours (before any subsequent administration) to highlight the possible presence of edema or irritation in the vaginal and perineal areas.

24 hours following the last administration, the rabbits have been sacrificed, the vaginal mucosa dissected in order to perform the histological examination of the tissue and to evaluate the possible irritation and the presence of lactobacilli among the vaginal epithelial cells.

The results are summarized in the following Tables 1 and 2.

**Table 1: Vaginal Irritation (Ir.I Irritation index)**

| Sample | | Macroscopic analysis | Microscopic analysis | Vaginal | |
|---|---|---|---|---|---|
| | TG | CG | TG | CG | |
| *L. Plantarum* P17630 Vaginal Capsule NV | nothing abnormal | nothing abnormal | Ir.I: 2.67 | Ir.I: 0.67 | 2.00 |

**Table 2: Adhesion**

| Sample | | *Lactobacillus Plantarum* P17630 presentamong the epithelial cells | | | |
|---|---|---|---|---|---|
| | | (CFU: colony-forming units) | | | |
| | | TG | | | CG |
| *L. Plantarum* P17630 Vaginal Capsule NV | | average 53 CFU | | | absent |
| | | (45; 56; 58 CFU) | | | |

The data set forth in Table 1 denote that the sample used in the TG can be classified as minimally irritant for the vaginal mucosa according to the "Vaginal Irritation Test ISO 10993-10:2010".

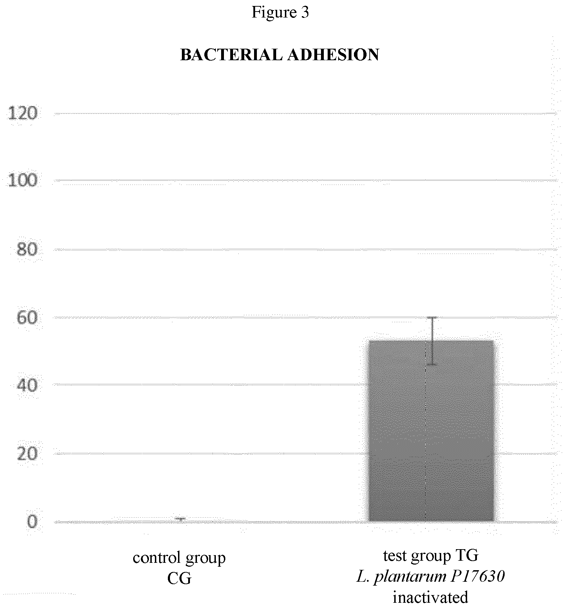

The data showed in Table 2 are schematized in Figure 3.

On the basis of the data set forth above, it is possible to conclude that the *Lactobacillus Plantarum* P17630 inactivated according to the invention and contained into the vaginal capsules used in the test, can adhere to the epithelial cells of the vaginal mucosa although it is not viable. This data confirms that the inactivation process, which allows to keep intact the cell wall of the Lactobacillus, does not affect its adhesion ability to the mucosa.

## Claims

1. A topical composition comprising at least one strain of at least one non-viable probiotic bacterium, optionally together with conventional excipients and/or carriers, said bacterium having a substantially intact cell wall.

2. The composition according to claim 1, **characterized in that** said at least one strain of at least one probiotic bacterium has, on its surface, molecules and/or molecular and/or macromolecular structures assigned to the adhesion.

3. The composition according to claim 1, **characterized in that** said molecules and/or molecular structures assigned to the adhesion are the adhesines.

4. The composition according to any one of claims 1 to 3, **characterized in that** said at least one strain of at least one probiotic bacterium is made non-viable by DNA impairment.

5. The composition according to claim 4, **characterized in that** at least one probiotic bacterium is made non-viable by gamma ray treatment.

6. The composition according to any one of claims 1 to 5, **characterized in that** said at least one strain of at least one probiotic bacterium is selected from the bifidobacterium, lactobacillus, streptococcus genera and mixtures thereof.

7. The composition according to claim 6, **characterized in that** said at least one strain of at least one probiotic bacterium is selected from *Lactobacillus salivarius* I 1794, *Lactobacillus paracasei* I 1688, *Lactobacillus paracasei* I 1687, *Lactobacillus fermentum* I 3677, filed at the CIP Institut Pasteur of Paris, France and *Lactobacillus plantarum* P 17630, *Lactobacillus crispatus* P 17631, *Lactobacillus gasseri* P 17632, *Lactobacillus gasseri* P 18137, *Lactobacillus acidophilus* P 18806, *Lactobacillus delbrueckii* P 18805, *Streptococcus thermophilus* P 18807, *Lactobacillus brevis* CV7Lac (P30019), *Lactobacillus paracasei* LC11 (P30020), *Lactobacillus brevis* Y1 (P30021), *Lactobacillus brevis* CV2Lac (P30022), *Lactobacillus plantarum* CVI15B (P30023), *Lactobacillus paracasei* LC18 (P30024), *Lactobacillus plantarum* LM2 (P30025), filed at the BCCM-LMG Laboratorium vor Microbiologie, University of Gent, Gent Belgium and *Lactobacillus rhamnosus* ATCC®53103™, filed at the American Type Culture Collection of Manassas, VA, USA and mixtures thereof.

8. The composition according to claim 7, **characterized in that** said at least one probiotic bacterium is the *Lactobacillus plantarum* P 17630.

9. The composition according to claim 7, **characterized in that** said at least one probiotic bacterium is selected from *Lactobacillus plantarum* P 17630, *Lactobacillus paracasei* I 1688, *Lactobacillus salivarius* I 1794, *Lactobacillus crispatus* P 17631 and mixtures thereof.

10. The topical composition according to any one of claims 1 to 9, **characterized in that** it is selected from pharmaceutical and/or cosmetic compositions such as capsules, suppositories, creams, multiple emulsions such as oil and/or silicone in water emulsions, water-in-oil and/or silicone emulsions, water/oil/water or water/silicone/water emulsions and oil/water/oil or silicone/water/silicone emulsions, anhydrous compositions, aqueous dispersions, oils, milk, balms, foams, lotions, gel, creme gel, hydroalcoholic solutions, hydroglicolic solutions, hydrogel, liniments, waxes, soaps, shampoos, emollients, serums, polysaccharide films, unguents, mousses, ointments, powders, sticking patches, non-sticking patches, occlusive patches, micro-electric patches, pastes, sprays, deodorants, sun products, moisturizing creams, anti-aging creams, anti-pollution creams, creams containing pigments, radiant creams, colored and non-colored powders for face and body, cream foundation, makeup remover lotions, makeup remover milks, concealers, mascara, eye shadows, lipsticks, lip balms and lip glosses.

11. The topical composition according to any one of claims 1 to 10 for its use in therapy.

12. The topical composition for the use according to claim 11, in the prevention and/or the treatment of skin diseases and mucosal diseases.

13. The topical composition for the use according to claim 11 or 12, in the prevention and/or the treatment of diseases caused by pathogenic microorganisms and/or of pityriasis versicolor and/or of dandruff caused by fungi of the *Malassezia* genus.

14. The composition for the use according to any one of claims 11 to 13, in the prevention and/or the treatment of vaginal diseases.

15. The composition for the use according to any one of claims 11 to 13, in the prevention and/or the treatment of oral mucosa diseases, preferably aphthae.
